# EUROPEAN PATENT SPECIFICATION

(11) **EP 2 059 201 B1**
(45) Date of publication and mention of the grant of the patent: **11.11.2015**
(21) Application number: 07814262.7
(22) Date of filing: 20.08.2007
(51) Int. Cl.: A61F 9/009, A61F 9/013, A61F 2/14, A61F 9/008

(54) **SYSTEM AND METHOD FOR RESECTING CORNEAL TISSUE**
SYSTEM UND VERFAHREN FÜR HORNHAUTGEWEBERESEKTION
SYSTÈME ET PROCÉDÉ DE RÉSECTION DU TISSU CORNÉEN

(30) Priority: 05.09.2006 US 469941
(43) Date of publication of application: 20.05.2009
(73) Proprietor: AMO Development, LLC, Santa Ana, CA 92705 (US)
(72) Inventor: KURTZ, Ronald, M., Irvine, CA 92603 (US); JOTYAN, Gagik, 2510 Dorog (HU); JUHASZ, Tibor, Irvine, CA 92620 (US); SARAYBA, Melvin, A., Ladera Ranch, CA 92694 (US)
(74) Representative: Clark, Jane Anne
(86) International application number: PCT/US2007/076334
(87) International publication number: WO 2008/030698

(56) References cited:
- EP-A2- 1 666 009
- WO-A1-2007/012924
- WO-A2-2007/143111
- US-A- 5 549 632
- US-A1- 2003 208 190
- US-A1- 2004 054 358
- US-A1- 2006 015 090
- US-A1- 2006 100 612
- US-B1- 6 325 792

## Description

### BACKGROUND OF THE INVENTION

The field of the present invention is systems for transplanting corneas.

Many different diseases or conditions of the cornea exist which completely or effectively rob those who suffer from such diseases or conditions of vision. Fortunately, corneal transplant procedures, which are becoming more commonplace, are capable of substantially restoring lost vision. Currently, there are many techniques and tools for performing corneal transplantation. Unfortunately, all suffer from inherent limitations or risk of complications.

A procedure called posterior lamellar keratoplasty (PLK), or deep lamellar keratoplasty, was previously developed to address these shortcomings for patients with endothelial cell dysfunction. In this procedure, a thin posterior or lenticule of stromal tissue (along with descemets membrane and endothelial cells attached) is removed from the cornea of a diseased eye. A similar procedure is performed on a donor eye to obtain donor tissue. When the donor tissue is placed in the recipient's eye, no sutures are required because the anterior surface of the recipients' cornea was left intact. One advantage of this procedure is that it results in little post-operative astigmatism. While PLK has its advantages, in practice it is difficult to perform, regardless of whether manual or automated instruments are used.

A procedure called descemets strip endokeratoplasty was introduced in attempts have been made to improve on PLK. In this procedure, the stromal tissue is not removed from the recipient's cornea, rather only the descemets membrane and endothelial cells are removed. While this simplifies the procedure, it results in an abnormally thick post-operative cornea and may degrade optical quality. In addition, there is the added risk of the additional tissue becoming dislodged from the recipient's cornea since no cavity was created into which it can be fully inserted.

Other attempts have been made to improve on PLK using femtosecond lasers to incise both the recipient's and donor's cornea with high precision. While this makes PLK more practical, it also introduces some new difficulties into the procedure. Specifically, contact lenses, both flat and curved, are generally used with femtosecond lasers in order to provide proper registration of the cornea with the focal point of the pulsed laser beam. These contact lenses distort the shape of the cornea by forcing the anterior surface of the cornea to conform to the curvature of the lens. This distortion of the cornea introduces folds or wrinkles into the corneal tissue, with the folds being more pronounced in the posterior portion of the cornea. Thus, when a resection incision is made, the folds or wrinkles can cause the incision to have an irregular surface when the cornea returns to its natural curvature if the incision is made too near the posterior surface of the cornea. Such an irregular surface, however, can have adverse effects on wound healing and optical quality of the post-operative cornea.

Another problem arises when the resection incision is made too near the anterior surface of the cornea. Under such circumstances, the surgically repaired cornea may begin to weaken and deteriorate due to ectasia because the anterior portion was overly thinned during the procedure.

EP 1,666,009 describes a system for refractive ophthalmic surgery in which a flap of cornea is cut and then lifted to expose the underlying corneal tissue and the underlying corneal tissue is then reshaped by photoablation.

US 2006/0015090 describes a corneal refraction procedure providing a customised transition zone to provide continuous curvature between an ablated optical zone and a non-ablated optical zone to address curvature discontinuity.

US 2006/0100612 describes a laser-based device for non-mechanical three-dimensional trepanation during corneal transplants.

US 6,325,792 describes the application of low energy ultra-short (femtosecond) pulsed laser radiation to a patient's eye in one of a number of patterns such that the exposed ocular tissue is ablated or excised through the process of optical breakdown or photodisruption.

US 2007/012924 which published after the priority date of the present application and so can therefore be considered only for the purposes of novelty under Article 54(3) EPC describes a system and method for compensating a corneal dissection in which pre-dissection diagnostic information about the transparent material to be dissected is used to define a topology and is analysed to calculate a predicated result of a dissection along a prototypic dissection path.

### BRIEF SUMMARY OF THE INVENTION

The present invention is set out in the appended claims. Described herein are a system and method for resecting corneal tissue. In the system, a surgical laser emits a pulsed laser beam which is directed into the cornea by a focusing assembly. An interface is programmed with a biomechanical model of corneal tissue, which is used to provide a depth range for selection of a resection depth from an anterior corneal surface. The selected resection depth is received by a controller which employs the focusing assembly to move the focal point of the pulsed laser beam and make a resection incision at the resection depth for resecting a portion of the cornea.

In the method, the resection depth is first determined using a biomechanical model of the cornea. Thereafter, a resection incision is made at the resection depth for purposes of resecting a posterior portion of the donor cornea.

Other options may be added to the above system or process, either singly or in combination. In a first option, the resection depth is determined using the anterior surface of the cornea as a reference. In a second option, a contact lens is placed against the anterior surface of the cornea. The contact lens is adapted to conform the anterior surface of the cornea to the shape of the contact lens, which preferably has a radial or planar form. With the contact lens in place, the resection incision is made using a surgical laser. In a third option, the resection depth is selected to minimize surface irregularities resulting from the resection incision. In a fourth option, the resection depth is selected to minimize post-operative weakening of the cornea. In a fifth option, the resection incision is placed at a unifonn distance fi-om one of the posterior surface of the cornea or the anterior surface of the cornea. In a sixth option, a posterior portion of corneal tissue is resected from a donor cornea, and this donor tissue is grafted into the recipient cornea. In resecting the donor tissue, the ratio of the depth of the resection incision in the recipient cornea, as compared to the overall thickness of the recipient cornea, is first determined. From this ratio, the depth of the resection incision in the donor cornea may be determined. This is done by making the depth of the resection incision in the donor cornea have the same ratio as compared to the overall thickness of the donor cornea.

Accordingly, an improved system and method of resecting corneal tissue are disclosed. Advantages of the improvements will appear from the drawings and the description of the preferred embodiment.

### BRIEF DESCRIPTION OF THE DRAWINGS

In the drawings, wherein like reference numerals refer to similar components:
Fig. 1 is a flow chart illustrating steps for performing a corneal transplant procedure;
Figs. 2A & 2B illustrate resection incisions in a recipient cornea and a donor cornea, respectively, as part of a corneal transplant procedure; and
Fig. 3 is a schematic view of a system for resecting corneal tissue.

### DETAILED DESCRIPTION OF THE INVENTION

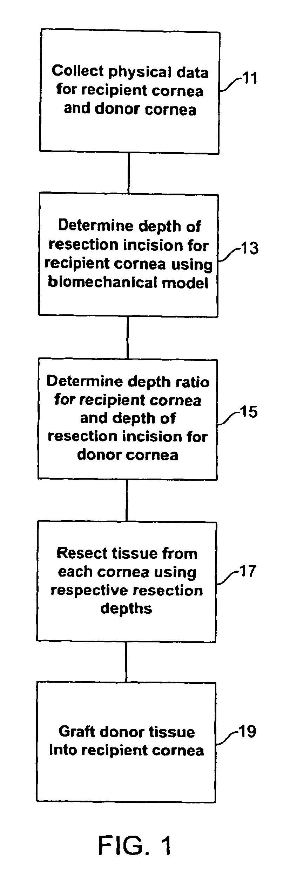

Turning in detail to the drawings, Fig. 1 is a flow chart illustrating a process for performing a PLK procedure. The basic steps of the PLK procedure are (1) collect physical data 11 for both the recipient cornea and the donor cornea; (2) determine the depth of the resection incision 13 for the recipient cornea; (3) determine the depth ratio of the resection incision 15 in the recipient cornea and the depth of the resection incision in the donor cornea; (4) resect tissue 17 from both the recipient and donor corneas; and (5) graft 19 the donor tissue into the recipient cornea. Each step is explained in further detail below.

The physical data collected includes thickness measurements of both the recipient and donor corneas. These thickness measurements are used to develop a thickness profile of each cornea. Additional physical data may also be collected for each cornea, with the type of data collected being dependent upon the requirements of biomechanical model used in the subsequent step of the PLK procedure. This thickness profile, along with any other data needed for the biomechanical model, may be obtained by any one of the many known methods for measuring the physical structure of the eye, with the preferred method being through optical coherence tomography (OCT). Many commercially available OCT scanners are capable of performing such measurements. One example is the VisanteTM OCT scanning system, manufactured by Carl Zeiss Meditec, which has an office in Dublin, California. One advantage of the VisanteTM OCT system is that it does not make contact with the cornea when performing the OCT scan.

Following collection of the physical data, the depth of the resection incision for the recipient cornea is determined using the biomechanical model. Preferably, the biomechanical model takes into account stresses introduced into the corneal tissue when the cornea is conformed to the shape of a contact lens set against the anterior surface when the resection incision is made with a surgical laser system. Such stresses can cause folds in the corneal tissue which are more pronounced in the posterior regions of the cornea. A resection incision made in areas with more pronounced folds can lead directly to surface irregularities at the resection incision when contact lens is removed and the cornea returns to its normal shape. Therefore, the biomechanical model is used to place the resection incision at a depth which minimizes or eliminates surface irregularities at the resection incision.

The biomechanical model also preferably takes into account the post-operative stability of the recipient cornea when determining the depth of the resection incision. Without sufficient post-operative stability, the cornea may undergo weakening through pathological deterioration, e.g., ectasia, thereby exposing the recipient to a risk of vision impairment. Maintaining sufficient tissue between the anterior surface of the recipient cornea and the resection incision is an important factor to maintaining post-operative stability. The biomechanical model is therefore employed to determine the minimum depth from the anterior surface at which the resection incision is likely to cause post-operative stability problems.

Optionally, the biomechanical model may be developed to employ a database of corneal measurements collected from many patients. Using information from the database, together with at least the known curvature of the contact lens, such a biomechanical model could be used to determine an appropriate depth for the resection incision.

The resection depth selected for the recipient cornea is selected to be between the minimum depth needed to create post-operative stability in the recipient cornea and an appropriate depth to avoid or eliminate surface irregularities at the resection incision. In the event that there is no region between the two extremes, maintaining post-operative stability in the cornea is preferred. In such instances, the radius of the contact lens upon which the shape of the cornea is conformed for purposes of the PLK procedure and the biomechanical model could be enlarged to reduce stresses on the corneal tissue and help create a region in which post-operative stability can be maintained along with minimizing irregularities at the resection incision.

The biomechanical model may be one of several previously developed models which are known to skilled artisans, or it may be one which is specifically developed for the PLK procedure. Modeling software, such as the software published by Structural Research & Analysis Corp. of Santa Monica, CA under the title "Cosmos/M", may be used to develop biomechanical models. One such model is described in the article by G. Djotyan et al., "Finite Element Modeling of Posterior Lamellar Keratoplasty: Construction of Theoretical Nomograms for Induced Refractive Errors", Ophthalmic Research, Vol.38, n.5, 2006.

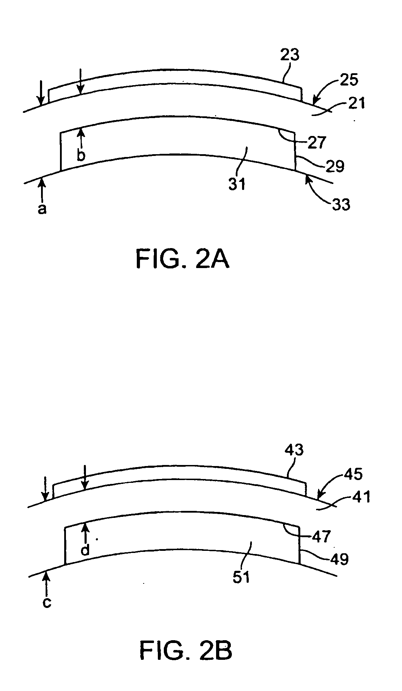

Fig. 2A illustrates the recipient cornea 21 with a contact lens 23 placed against the anterior surface 25 of the recipient cornea 21. The resection incision 27 and the sidecut 29 are made to remove a posterior portion of corneal tissue 31 in preparation for the transplant. The resection incision 27 may be made using several different techniques. However, it should be noted that not all techniques provide the same quality of clinical results following the transplant. One technique is to make the resection incision 27 at a uniform distance from the posterior surface 33 of the cornea. U.S. Pat. App. No. 11/375,542, (US 2007/0219541) discloses a method of making such an incision. By way of further example, techniques for making the resection incision 27 at a uniform distance from the anterior surface 25 are disclosed in U.S. Patent No. 5,993,438, U.S. Patent No. 6,730,074, and U.S. Patent Publication No. 20050245915. Other techniques known to skilled artisans may also be employed.

The thickness of the recipient cornea 21 is expressed as 'a'. The depth, b, of the resection incision 27 shown in Fig. 1 is measured from the anterior surface 25 of the cornea. The ratio of the depth, b, to the thickness, a, is employed to determine the depth of the resection incision in the donor cornea as described below.

Similar to the resection incision 27, the sidecut 29 may also be formed through the different techniques that are known to skilled artisans.

The contact lens 23 is a rigid lens placed against the anterior surface 25, thereby forcing the anterior surface 25 to conform to the shape of the contact lens 23. Preferably, the contact lens 23 confirms the anterior surface 25 to a radial or planar shape. U.S. Patent No. 5,549,632, describes making a laser incision by deforming the shape of the cornea. U.S. Patent No. 6,863,667 and U.S. Pat. App. No. 11/258,399, (US 2007-0093796) describe patient interface devices which may be used to align the surgical laser with the recipient cornea for purposes of making accurate incisions. Of course, the contact lens is not needed if the surgical laser system is capable of alignment with sufficient precision without use of the contact lens.

Fig. 2B illustrates the donor cornea 41 with a contact lens 43 placed against the anterior surface 45 of the donor cornea 41. The resection incision 47 and the sidecut 49 are made to remove the donor tissue 51 for grafting into the recipient cornea. The resection incision 47 and the sidecut 49 are preferably made using the same techniques employed to incise the recipient cornea. The ratio of the depth, d, of the resection incision 47 to the thickness, c, of the donor cornea 41 is preferably the same as the ratio that was set for the recipient cornea. By setting the depth of the resection incision 47 in this manner, compensation is made in the event that the donor cornea 41 swells due to fluid absorption following removal from the donor eye. Such swelling occurs because the donor cornea is resected from the donor eye in facility separate from the one in which the transplant is performed.

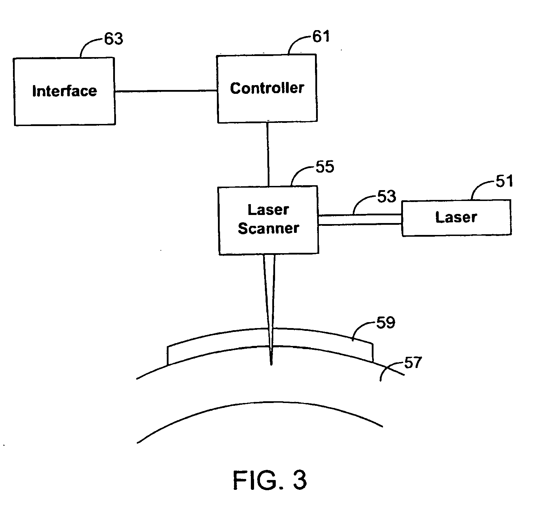

Referring to Fig. 3, a surgical system is shown which may be used to incise both a donor cornea or a recipient cornea. A femtosecond surgical laser 51 generates a pulsed laser beam 53 and directs that beam into the focusing assembly 55, which in turn focuses the pulsed beam 53 into the cornea 57. A contact lens 59 is placed over the cornea to deform the anterior corneal surface as described above. The controller 61 is a programmable computer which precisely controls the location of the beam focal point within the cornea 57 according to parameters received from the programmable surgeon interface 63. The interface 63 is programmed with a biomechanical model of the cornea and presents the surgeon with an incision depth range within which the surgeon may select the resection depth. The resection depth is received by the controller 61, which uses the focusing assembly 55 to make the resection incision at the resection depth.

A pulsed laser beam having ultra-short pulses, preferably in the femtosecond range, is employed to make the incisions. The laser may be of the type described in U.S. Patent No. 4,764,930, producing an ultra-short pulsed beam as described in one or both of U.S. Patent No. 5,984,916 and U.S. Patent No. RE37,585.

Commercial lasers capable of performing the incisions are available from IntraLase Corp. of Irvine, California.

Thus, a system and method of resecting corneal tissue are disclosed. While embodiments of this invention have been shown and described, it will be apparent to those skilled in the art that many more modifications are possible without departing from the inventive concepts herein. The invention, therefore, is not to be restricted except by the following claims.

## Claims

1. A system for resecting corneal tissue, the system comprising:
an interface (63) programmed with a biomechanical model of corneal tissue, the interface being adapted to provide a depth range using the biomechanical model for selection of a resection depth from an anterior corneal surface;
a surgical laser (51) adapted to emit a pulsed laser beam;
a focusing assembly (55) adapted to focus the pulsed laser beam into a cornea;
a controller (61) adapted to receive the resection depth from the interface, to move a focal point of the pulsed laser beam within a cornea (57) using the focusing assembly, and to direct the focal point of the pulsed laser beam to make a resection incision (27; 47) for resecting a portion of the cornea, wherein the resection incision is made at the resection depth (b; d).

2. The system of claim 1 further comprising:
a contact lens (23; 43) adapted to be placed against the anterior surface and conform the anterior surface to a shape of the contact lens, wherein the biomechanical model accounts for deformation of the cornea (57).

3. The system of claim 2, wherein the contact lens (23; 43) applanates the anterior surface.

4. The system of claim 1, wherein the biomechanical model is based on a thickness profile of the cornea and stresses introduced into the cornea associated with conforming the cornea to the shape of a contact lens (23; 43) and wherein the depth range is provided by the biomechanical model to minimize surface irregularities at the resection incision (27; 47).

5. The system of claim 1, wherein the biomechanical model is based on a thickness profile of the cornea and stresses introduced into the cornea associated with conforming the cornea to the shape of a contact lens (23; 43) and wherein the depth range is provided by the biomechanical model to minimize post-operative weakening of the cornea.

6. The system of claim 1, wherein the resection incision is at a uniform distance from one of a posterior surface of the cornea (57) or the anterior surface.

7. The system of claim 1, wherein the controller is adapted to make the resection incision for resecting a posterior portion of the cornea (57).

8. The system of claim 1, wherein the resection depth is between a first depth based on the biomechanical model and a second depth based on the biomechanical model, the first depth corresponding to maintaining a post-operative stability of the recipient cornea, the second depth corresponding to minimizing surface irregularities at the resection depth associated with a resection incision (27) in the recipient corneal while the recipient cornea is conformed to the shape of a contact lens (23).

9. A method of resecting donor corneal tissue from a donor cornea, the method comprising:
determining a resection depth from an anterior surface of the donor cornea using a corneal biomechanical model; and
making a donor resection incision (47) for resecting a donor posterior portion from the donor cornea, wherein the resection incision (47) is made at the resection depth (d).

10. A method according to claim 9 of resecting corneal tissue from a donor cornea for transplanting to a recipient cornea, wherein determining the resection depth comprises:
determining the recipient resection depth from a recipient anterior surface of a recipient cornea using a biomechanical model of the recipient cornea.

11. A method according to claim 9 or 10 wherein the resection depth is between a first depth based on the biomechanical model and a second depth based on the biomechanical model, the first depth corresponding to maintaining a post-operative stability of the recipient cornea, the second depth corresponding to minimizing surface irregularities at the resection depth associated with a resection incision (27) in the recipient corneal while the recipient cornea is conformed to the shape of a contact lens (23).

## Patentansprüche

1. System zur Hornhautgeweberesektion, wobei das System Folgendes aufweist:
eine Schnittstelle (63), die mit einem biomechanischen Modell von Hornhautgewebe programmiert ist, wobei die Schnittstelle zum Bereitstellen eines Tiefenbereiches unter Verwendung des biomechanischen Modells zur Auswahl einer Resektionstiefe von einer Hornhautvorderfläche geeignet ist;
einen chirurgischen Laser (51), der zum Aussenden eines gepulsten Laserstrahls geeignet ist;
eine Fokussierbaugruppe (55), die zum Fokussieren des gepulsten Laserstrahls in eine Hornhaut geeignet ist;
einen Controller (61), der zum Empfangen der Resektionstiefe von der Schnittstelle, zum Bewegen eines Brennpunktes des gepulsten Laserstrahls innerhalb einer Hornhaut (57) unter Verwendung der Fokussierbaugruppe und zum Lenken des Brennpunktes des gepulsten Laserstrahls zum Vornehmen eines Resektionseinschnittes (27; 47) zum Resezieren eines Abschnittes der Hornhaut geeignet ist, wobei der Resektionseinschnitt mit der Resektionstiefe (b; d) vorgenommen wird.

2. System nach Anspruch 1, welches ferner Folgendes aufweist:
eine Kontaktlinse (23; 43), die gegen die Vorderfläche platziert werden kann und zum Anpassen der Vorderfläche an eine Form der Kontaktlinse geeignet ist, wobei das biomechanische Modell eine Verformung der Hornhaut (57) berücksichtigt.

3. System nach Anspruch 2, wobei die Kontaktlinse (23; 43) die Vorderfläche verflacht.

4. System nach Anspruch 1, wobei das biomechanische Modell auf einem Dickenprofil der Hornhaut und Spannungen, die im Zusammenhang mit dem Anpassen der Hornhaut an die Form einer Kontaktlinse (23; 43) in die Hornhaut eingebracht werden, basiert und wobei der Tiefenbereich durch das biomechanische Modell bereitgestellt wird, um Oberflächenunregelmäßigkeiten am Resektionseinschnitt (27; 47) zu minimieren.

5. System nach Anspruch 1, wobei das biomechanische Modell auf einem Dickenprofil der Hornhaut und Spannungen, die im Zusammenhang mit dem Anpassen der Hornhaut an die Form einer Kontaktlinse (23; 43) in die Hornhaut eingebracht werden, basiert und wobei der Tiefenbereich durch das biomechanische Modell bereitgestellt wird, um eine postoperative Schwächung der Hornhaut zu minimieren.

6. System nach Anspruch 1, wobei sich der Resektionseinschnitt in einer gleichmäßigen Entfernung von entweder einer Rückfläche der Hornhaut (57) oder der Vorderfläche befindet.

7. System nach Anspruch 1, wobei der Controller zum Vornehmen des Resektionseinschnittes zum Resezieren eines Rückabschnittes der Hornhaut (57) geeignet ist.

8. System nach Anspruch 1, wobei die Resektionstiefe zwischen einer ersten Tiefe basierend auf dem biomechanischen Modell und einer zweiten Tiefe basierend auf dem biomechanischen Modell liegt, wobei die erste Tiefe dem Aufrechterhalten einer postoperativen Stabilität der Empfängerhornhaut entspricht und die zweite Tiefe dem Minimieren von Oberflächenunregelmäßigkeiten bei der Resektionstiefe im Zusammenhang mit einem Resektionseinschnitt (27) in der Empfängerhornhaut entspricht, während die Empfängerhornhaut an die Form einer Kontaktlinse (23) angepasst wird.

9. Verfahren zum Resezieren von Spenderhornhautgewebe von einer Spenderhornhaut, wobei das Verfahren Folgendes aufweist:
Bestimmen einer Resektionstiefe von einer Vorderfläche der Spenderhornhaut unter Verwendung eines biomechanischen Hornhautmodells; und
Vornehmen eines Spender-Resektionseinschnittes (47) zum Resezieren eines Spender-Rückabschnittes von der Spenderhornhaut, wobei der Resektionseinschnitt (47) mit der Resektionstiefe (d) erfolgt.

10. Verfahren nach Anspruch 9 zum Resezieren von Hornhautgewebe aus einer Spenderhornhaut zum Transplantieren in eine Empfängerhornhaut, wobei das Bestimmen der Resektionstiefe Folgendes aufweist:
Bestimmen der Empfänger-Resektionstiefe von einer Empfänger-Vorderfläche einer Empfängerhornhaut unter Verwendung eines biomechanischen Modells der Empfängerhornhaut.

11. Verfahren nach Anspruch 9 oder 10, wobei die Resektionstiefe zwischen einer ersten Tiefe basierend auf dem biomechanischen Modell und einer zweiten Tiefe basierend auf dem biomechanischen Modell liegt, wobei die erste Tiefe dem Aufrechterhalten einer postoperativen Stabilität der Empfängerhornhaut entspricht und die zweite Tiefe dem Minimieren von Oberflächenunregelmäßigkeiten bei der Resektionstiefe im Zusammenhang mit einem Resektionseinschnitt (27) in der Empfängerhornhaut entspricht, während die Empfängerhornhaut an die Form einer Kontaktlinse (23) angepasst wird.

## Revendications

1. Système pour réséquer du tissu cornéen, le système comprenant :
une interface (63) programmée avec un modèle biomécanique de tissu cornéen, l'interface étant conçue pour fournir une plage de profondeurs au moyen du modèle biomécanique pour une sélection de profondeurs de résection à partir d'une surface cornéenne antérieure ;
un laser chirurgical (51) conçu pour émettre un faisceau laser à impulsion ;
un assemblage de focalisation (55) conçu pour focaliser le faisceau laser à impulsion dans une cornée ;
un contrôleur (61) conçu pour recevoir la profondeur de résection depuis l'interface, pour déplacer un point de focalisation du faisceau laser à impulsion dans une cornée (57) au moyen de l'assemblage de focalisation, et pour diriger le point de focalisation du faisceau laser à impulsion pour réaliser une incision de résection (27 ; 47) pour réséquer une partie de la cornée, où l'incision de résection est réalisée à la profondeur de résection (b ; d).

2. Système selon la revendication 1, comprenant en outre :
une lentille de contact (23 ; 43) conçue pour être placée contre la surface antérieure et pour conformer la surface antérieure à une forme de la lentille de contact, où le modèle biomécanique rend compte de la déformation de la cornée (57).

3. Système selon la revendication 2, dans lequel la lentille de contact (23 ; 43) aplanit la surface antérieure.

4. Système selon la revendication 1, dans lequel le modèle biomécanique est basé sur un profil d'épaisseur de la cornée et sur des contraintes introduites dans la cornée associées à une mise en conformité de la cornée à la forme de la lentille de contact (23 ; 43) et où la plage de profondeurs est fournie par le modèle biomécanique pour minimiser des irrégularités de surface au niveau de l'incision de résection (27 ; 47).

5. Système selon la revendication 1, dans lequel le modèle biomécanique est basé sur un profil d'épaisseur de la cornée et sur des contraintes introduites dans la cornée associées à une mise en conformité de la cornée à la forme de la lentille de contact (23 ; 43) et où la plage de profondeurs est fournie par le modèle biomécanique pour minimiser des faiblesses postopératoires de la cornée.

6. Système selon la revendication 1, dans lequel l'incision de résection se situe à une distance uniforme d'une surface postérieure de la cornée (57) ou de la surface antérieure.

7. Système selon la revendication 1, dans lequel le contrôleur est conçu pour réaliser l'incision de résection pour réséquer une partie postérieure de la cornée (57).

8. Système selon la revendication 1, dans lequel la profondeur de résection se situe entre une première profondeur basée sur le modèle biomécanique et une seconde profondeur basée sur le modèle biomécanique, la première profondeur consistant à maintenir une stabilité postopératoire de la cornée du receveur, la seconde profondeur consistant à minimiser des irrégularités de surface au niveau de la profondeur de résection associées à une incision de résection (27) dans le cornée du receveur tandis que la cornée du receveur est conforme à la forme d'une lentille de contact (23).

9. Procédé pour réséquer un tissu de cornée de donneur à partir d'une cornée de donneur, le procédé comprenant les étapes suivantes :
déterminer une profondeur de résection à partir d'une surface antérieure de la cornée du donneur au moyen d'un modèle biomécanique cornéen ; et
réaliser une incision de résection du donneur (47) pour réséquer une partie postérieure du donneur à partir de la cornée du donneur, où l'incision de résection (47) est réalisée à la profondeur de résection (d).

10. Procédé selon la revendication 9, de résection de tissu cornéen à partir d'une cornée de donneur pour une transplantation vers une cornée de receveur, où la détermination de la profondeur de résection comprend l'étape suivants :
déterminer la profondeur de résection du receveur au moyen d'un modèle biomécanique de la cornée du receveur.

11. Procédé selon la revendication 9 ou la revendication 10, dans lequel la profondeur de résection se situe entre une première profondeur basée sur le modèle biomécanique et une seconde profondeur basée sur le modèle biomécanique, la première profondeur correspondant au maintien d'une stabilité postopératoire de la cornée du receveur, la seconde profondeur correspondant à la minimisation des irrégularités de surface au niveau de la profondeur de résection associées à une incision de résection (27) dans la cornée du receveur tandis que la cornée du receveur est conforme à la forme d'une lentille de contact (23).
